(19) Europäisches Patentamt
European Patent Office
Office européen des brevets

(11) Veröffentlichungsnummer : **0 025 838**
**B1**

(12) # EUROPÄISCHE PATENTSCHRIFT

(45) Veröffentlichungstag der Patentschrift : **15.12.82**

(51) Int. Cl.³ : **C 07 D307/88**

(21) Anmeldenummer : **80104455.3**

(22) Anmeldetag : **29.07.80**

(54) **Verfahren zur Herstellung von 3-Bromphthalid.**

(30) Priorität : **29.08.79 DE 2934822**

(43) Veröffentlichungstag der Anmeldung : **01.04.81 (Patentblatt 81/13)**

(45) Bekanntmachung des Hinweises auf die Patenterteilung : **15.12.82 Patentblatt 82/50**

(84) Benannte Vertragsstaaten : **BE DE FR GB NL**

(56) Entgegenhaltungen :
**DE A 2 652 299**
**DE A 2 721 142**
**DE A 2 753 512**
**HOUBEN-WEYL « Methoden der organischen Chemie » 4. Auflage, Band V/4 : « Halogenverbindungen » 1960, GEORG THIEME VERLAG, Stuttgart Seite 26**

(73) Patentinhaber : **DYNAMIT NOBEL AKTIENGESELLSCHAFT**
**Patentabteilung Postfach 1209**
**D-5210 Troisdorf, Bez. Köln (DE)**

(72) Erfinder : **Englaender, Fritz, Dr.**
**Flossweg 10**
**D-5300 Bonn 2 (DE)**

**EP 0 025 838 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Verfahren zur Herstellung von 3-Bromphthalid

3-Bromphthalid läßt sich nach mehreren in der Literatur beschriebenen Verfahren herstellen. Man kann einmal von Phthalid ausgeben, das in der Schmelze mit Brom umgesetzt wird (Organic Syntheses Vol. 23 (1943)) und 3-Bromphthalid in 83 %iger Ausbeute liefert. Bei dem zweiten Verfahren wird Phthalid mit N-Bromsuccinimid umgesetzt (Organic Syntheses Coll. Vol. V 145-147 (1973)) und ergibt 3-Bromphthalid in 75-81 %iger Ausbeute.

Außerdem ist es möglich, o-Toluylsäure mit Brom umzusetzen und in 87 %iger Ausbeute 3-Bromphthalid zu erhalten (DE-OS 27 21 142). Eine weitere Synthese für 3-Bromphthalid besteht in der Umhalogenierung von 3-Chlorphthalid mittels gasförmigem Bromwasserstoff (DE-PS 26 52 299). Man erreicht bei dieser Synthese eine Ausbeute von über 90 %. Die beiden ersten Verfahren haben den Nachteil, daß vom eingesetzten Brom nur die Hälfte ausgenutzt werden kann, da die andere Hälfte zu HBr bzw. Bromid (bei der Herstellung des N-Bromsuccinimid) reagiert. Bei der Bromierung von o-Toluylsäure gehen von 4 eingesetzten Bromatomen sogar 3 in Form von HBr verloren.

Die Umhalogenierung mit Bromwasserstoff vermeidet diese Nachteile, hat jedoch den Nachteil, daß HBr wasserfrei nicht so leicht zugänglich ist wie elementares Brom. Außerdem benötigt man besonders konstruierte Reaktoren, damit das HBr-Gas in der Schmelze des 3-Chlorphthalids dispergiert und eine hohe Umsatzgeschwindigkeit und HBr-Ausbeute erreicht wird.

Es wurde nun gefunden, daß mit Phosphortribromid eine vorteilhafte Umhalogenierung von 3-Chlorphthalid möglich ist.

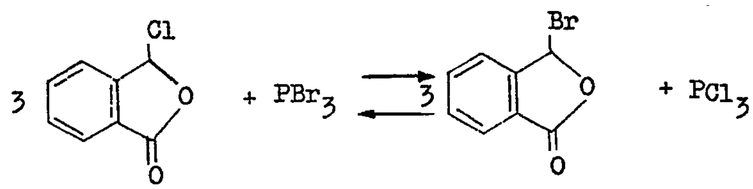


Gegenstand der Erfindung ist daher ein Verfahren zur Herstellung von 3-Bromphthalid durch Umhalogenierung von 3-Chlorphthalid, welches dadurch gekennzeichnet ist, daß man 3-Chlorphthalid mit mindestens der stöchiometrischen Menge Phosphortribromid umsetzt.

Es handelt sich um eine Gleichgewichtsreaktion. Durch Abdestillieren des niedrig siedenden Phosphortrichlorids (Kp. : 75 °C) wird das Gleichgewicht in Richtung des Zielproduktes verschoben.

Obwohl 1 Mol Phosphortribromid 3 Mol 3-Chlorphthalid umhalogenieren können, kommt man mit diesem Verhältnis oft nicht zu einem quantitativen Umsatz, da der Halogenaustausch stufenweise vor sich geht und folgende Gleichgewichte vorliegen :

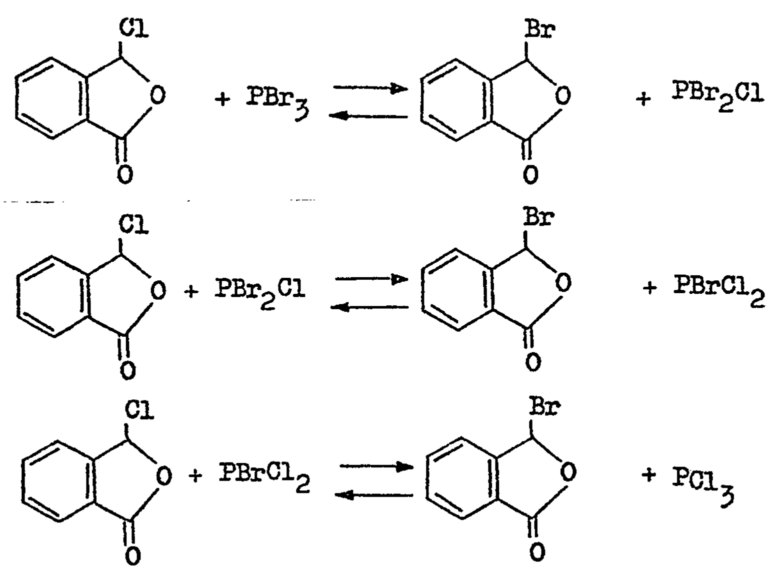


Beim Abdestillieren von $PCl_3$ gehen daher auch bromhaltige Phosphorchloride z.B. $PBrCl_2$ leicht mit über, so daß im Ansatz ein Bromdefizit entstehen kann und ein quantitativer Umsatz des 3-Chlorphthalids nicht mehr möglich ist.

Die zu verwendende Menge $PBr_3$ beträgt daher im allgemeinen das einfache bis sechsfache der

stöchiometrischen Menge, bevorzugt die dreifache bis sechsfache Menge, bezogen auf 3-Chlorphthalid. Die zunächst großerscheinende Menge des $PBr_3$ behindert jedoch die Reaktion keineswegs, stellt aber die Vollständigkeit der Reaktion sicher und vermeidet Reste von 3-Chlorphthalid, die nur schwer entfernbar sind.

Zudem kann $PBr_3$ nach der Abdestillation des $PCl_3$ ohne Mühe durch Destillation entfernt und von dem anschließend destillierten Produkt getrennt werden.

Das abdestillierte $PBr_3$ kann für weitere Umsetzungen eingesetzt werden. Kleine Mengen von chlorhaltigen Phosphorbromiden stören die Umsetzung nicht. Die bei der Reaktion verbrauchte Menge $PBr_3$ überschreitet daher die stöchiometrische Menge nicht oder nicht wesentlich.

Allgemein kann die Reaktion bei Temperaturen von etwa 50 bis 170 °C ausgeführt werden. Die Drucke liegen allgemein im Bereich von 2 mbar bis etwa 5 bar. Entschieden bevorzugt ist die Reaktion bei Normaldruck und die Vervollständigung der Reaktion bei Unterdruck.

Die Reaktion wird sehr bevorzugt ohne Anwesenheit von Lösungsmitteln oberhalb des Schmelzpunktes (65 bis 70 °C) des Chlorphthalids ausgeführt. Gegebenenfalls möglich, aber unzweckmäßig, sind Lösungsmittel, die sich bei der Reaktion inert verhalten und Chlorphthalid und Bromphthalid wenigstens teilweise lösen, beispielsweise aliphatische Kohlenwasserstoffe wie Petroläther, halogenierte aliphatische Kohlenwasserstoffe wie Tetrachlorkohlenstoff.

Soweit Lösungsmittel anwesend sind, kann die niedrigste Reaktionstemperatur bei etwa 50 °C liegen.

Es ist bevorzugt, die Reaktion bei Normaldruck zu beginnen und soweit zu vervollständigen, wie dieses bei Anwesenheit von $PCl_3$ möglich ist. Die Temperaturen liegen dann unterhalb der Siedetemperatur von $PBr_3$. Möglich aber unzweckmäßig ist geringer Überdruck bis etwa 5 bar und entsprechend höhere Reaktionstemperaturen. Anschließend wird der Druck gesenkt und die Temperatur erhöht, um zunächst $PCl_3$ abzudestillieren und die Reaktion zu vervollständigen und anschließend durch verstärktes Vakuum und im allgemeinen einer weiteren Erhöhung der Temperatur das getrennt aufzufangende $PBr_3$ und gegebenenfalls $PBrCl_2$ und $PBr_2Cl$ abzudestillieren. Im allgemeinen ist diese Entfernung der Phosphorhalogenide bei etwa 40 bis 20 mbar und 120 bis 155 °C beendet.

Die nachfolgende Vakuumdestillation des Produkts kann beispielsweise bei 20 bis 2 mbar und 120 bis 160 °C erfolgen und liefert ein Produkt guter Reinheit.

Das für die Reaktion benötigte Phosphortribromid braucht nicht als solches zugesetzt werden, sondern kann im Reaktionsgemisch aus rotem Phosphor und Brom erzeugt werden. Dadurch vereinfacht sich die Durchführung der Umhalogenierung ganz erheblich. Elementares Brom ist zudem leichter zugänglich als Bromverbindungen. Phosphor und Brom ist etwa in dem durch $PBr_3$ gegebenen stöchiometrischen Verhältnis oder einem Überschuß des Phosphors bis etwa 2 % zu verwenden, braucht jedoch vollständig erst am Ende der Reaktion vorzuliegen, so daß beispielsweise der Phosphor am Beginn oder ggf. in Portionen im Laufe der Reaktion zugegeben werden kann und Brom beispielsweise im Maße des Reaktionsfortschritts zugefügt wird.

Mit besonderem Vorteil kann also so vorgegangen werden, daß man zu einer Suspension von 3-Chlorphthalid und rotem Phosphor bei einer Temperatur, die oberhalb des Schmelzpunktes von 3-Chlorphthalid liegt, also oberhalb 65 bis 70 °C, die entsprechende Menge Brom zutropft. Brom setzt sich mit dem Phosphor in exothermer Reaktion zu Phosphortribromid um, so, daß ein Heizen des Reaktionsgemisches nicht mehr erforderlich ist. Sobald das Brom zugegeben ist werden die Phosphorhalogenide im Vakuum abdestilliert. Zum Schluß destilliert reines 3-Bromphthalid.

Die Aufarbeitung erfolgt vorzugsweise durch Destillation. Es ist jedoch auch möglich, das 3-Bromphthalid nach beendeter Umsetzung durch Kristallisation (z.B. aus Petroläther oder Tetrachlorkohlenstoff) zu reinigen.

3-Bromphthalid wird beispielsweise als Reagenz zur Einführung des Phthalidylrestes in organische Verbindungen, z.B. Antibiotika (DE-OS 22 28 255) verwendet.

## Beispiel 1

In einem Kolben werden 84,3 g 3-Chlorphthalid und 270,7 g Phosphortribromid gegeben und 4 Stunden auf 70 °C erhitzt. Dann wird das Gemisch im Vakuum destilliert, bis bei einem Druck von 20 mbar und einer Sumpftemperatur von 150 °C nicht mehr übergeht. Darauf wird bei 3 mbar destilliert. Bei 150 bis 155 °C erhält man 96,9 g Destillat entsprechend 91 % der theoretischen Menge. Der Schmelzpunkt des Destillats beträgt 66-80 °C. Nach der Umkristallisation erhält man 90,2 g (84,7 % der Theorie) reines 3-Bromphthalid vom Schmelzpunkt 84-85 °C.

## Beispiel 2

Zu einer Mischung von 84,3 g 3-Chlorphthalid und 31,3 g rotem Phosphor wird bei 70 °C 239,7 g Brom zugetropft. Danach hält man noch 4 Stunden bei 70 °C.

Nach der Aufarbeitung durch Vakuumdestillation erhält man 93,4 g 3-Bromphthalid (87,7 % der Theorie) mit der durch Gaschromatographie bestimmten Reinheit von 97,7 %.

Beispiel 3

Beispiel 2 wird wiederholt, jedoch wird die Menge des roten Phosphors auf 20,9 g, die Menge des zugetropften Broms auf 159,8 g gesenkt. Durch Destillation werden 90,5 g Produkt mit einer Reinheit von 95,9 % erhalten.

**Ansprüche**

1. Verfahren zur Herstellung von 3-Bromphthalid durch Umhalogenierung von 3-Chlorphthalid, dadurch gekennzeichnet, daß man 3-Chlorphthalid mit mindestens der stöchiometrischen Menge Phosphortribromid umsetzt.

2. Verfahren nach Anspruch 1, dadurch gekennzeichnet, daß die Menge der Phosphortribromide das einfache bis sechsfache der stöchiometrischen Menge beträgt.

3. Verfahren nach einem der Ansprüche 1 oder 2, dadurch gekennzeichnet, daß man Phosphortribromid im Reaktionsgemisch aus Brom und rotem Phosphor herstellt.

4. Verfahren nach mindestens einem der Ansprüch 1 bis 3, dadurch gekennzeichnet, daß die Reaktion durch Abdestillieren des gebildeten Phosphortrichlorids vervollständigt wird.

**Claims**

1. Process for the preparation of 3-bromophthalide by transhalogenation of 3-chlorophthalide, characterised in that 3-chlorophthalide is reacted with at least the stoichiometric amount of phosphorus tribromide.

2. Process according to claim 1, characterised in that the amount of phosphorus tribromide amounts to 1 to 6 times the stoichiometric amount.

3. Process according to one of claims 1 or 2, characterised in that phosphorus tribromide is prepared in the reaction mixture from bromine and red phosphorus.

4. Process according to at least one of claims 1 to 3, characterised in that the reaction is completed by distilling off the phosphorus trichloride formed.

**Revendications**

1. Procédé pour la préparation de 3-bromophtalide par transhalogénation du 3-chlorophtalide, caractérisé en ce que l'on fait réagir du 3-chlorophtalide avec au moins la quantité stœchiométrique de tribromure de phosphore.

2. Procédé selon la revendication 1, caractérisé en ce que la quantité de tribromure de phosphore est de une à six fois la quantité stœchiométrique.

3. Procédé selon l'une des revendications 1 ou 2, caractérisé en ce que l'on prépare le tribromure de phosphore dans le mélange de réaction à partir de brome et de phosphore rouge.

4. Procédé selon au moins l'une des revendications 1 à 3, caractérisé en ce que la réaction est complétée par distillation du trichlorure de phosphore formé.